# FASCICULE DE BREVET EUROPEEN

(11) **EP 1 629 118 B1**
(45) Date de publication et mention de la délivrance du brevet: **20.09.2006**
(21) Numéro de dépôt: 04742806.5
(22) Date de dépôt: 21.05.2004
(51) Int. Cl.: C12Q 1/68, C12N 9/12, C12P 19/34

(54) **COMPOSITION CONTENANT DES ACIDES NUCLEIQUES LYOPHILISES EN PRESENCE DE COLLAGENE ET DE DISACCHARIDES**
IN GEGENWART VON COLLAGEN UND DISACCHARIDEN LYOPHILISIERTE NUKLEINSÄUREN UMFASSENDE ZUSAMMENSETZUNG
COMPOSITION COMPRISING NUCLEIC ACIDS LYOPHILISED IN THE PRESENCE OF COLLAGEN AND DISACCHARIDES

(30) Priorité: 23.05.2003 FR 0306285
(43) Date de publication de la demande: 01.03.2006
(73) Titulaire: Robert, Raymond, 49240 Avrille (FR); Buffard, Claude, 94300 Vincennes (FR)
(72) Inventeur: Robert, Raymond, 49240 Avrille (FR); Buffard, Claude, 94300 Vincennes (FR)
(74) Mandataire: Vaillant, Jeanne
(86) Numéro de dépôt international: PCT/FR2004/001266
(87) Numéro de publication internationale: WO 2004/106549

(56) Documents cités:
- FR-A- 2 674 253
- US-A- 5 310 885
- US-A- 5 834 254
- US-A- 6 153 412
- US-A1- 2002 064 536

## Description

La présente invention concerne une méthode d'obtention d'une composition contenant des acides nucléiques par lyophilisation d'une solution aqueuse d'acides nucléiques en présence de disaccharides et de collagène, de préférence de tréhalose et de collagène. Elle porte également sur les compositions lyophilisées contenant des acides nucléiques, notamment des mélanges lyophilisés « prêt à l'emploi » contenant des composants nécessaires à l'amplification des acides nucléiques par PCR.

Les acides nucléiques et plus particulièrement les acides désoxyribonucléiques sont utilisés dans de nombreuses applications de biologie moléculaire, notamment dans les milieux réactionnels pour la réaction de polymérisation en chaîne (*Polymerase Chain Reaction,* PCR), le séquençage ou la transcription inverse (*Reverse Transcription,* RT).

En particulier, la réaction de polymérisation en chaîne (ci-après désignée par le terme « PCR ») permet d'amplifier une séquence d'ADN jusqu'à un million de fois et de détecter ainsi des séquences présentes dans un échantillon en très faible quantité, détectable après amplification.

Pour la mise en oeuvre d'une réaction PCR, des oligonucléotides capables d'hybrider aux extrémités de la séquence à amplifier sont préparés, en général par synthèse chimique. Ces oligonucléotides servent d'amorces pour la synthèse *in vitro* d'ADN catalysée par une ADN polymérase thermostable en présence de 2'-désoribonucléoside-5'-triphosphate. Chaque cycle de la PCR requiert une étape de dénaturation de l'ADN à amplifier, une étape d'hybridation des amorces et une étape d'élongation.

Pour obtenir une amplification suffisante, la PCR comprend en général de 25 à 40 cycles.

La PCR est décrite plus en détail, en particulier dans les brevets US 4,683,195, US 4,683,202 et US 4,800,159. Différentes méthodes alternatives ont également été décrites dans l'état de la technique incluant notamment les méthodes d'amplification isotherme telles que la TMA (transcription mediated amplification), la NASBA (nucleic acid sequence based amplification), la SSR (self sustained sequence replication) ou l'amplification par déplacement de brin (strand displacement amplification). Il est par ailleurs possible de combiner préalablement à la PCR, une réaction de transcription inverse (RT-PCR). La réaction de transcription inverse permet de transcrire un ARN messager en ADN complémentaire (ADNc) à l'aide d'une transcriptase inverse (*reverse transcriptase*).

Il est connu que les acides nucléiques, et en particulier les dNTPs ou les oligonucléotides sont des produits peu stables en solution et sont rapidement dénaturés lorsqu'ils sont conservés en solution à température ambiante. Pour ces raisons, les oligonucléotides et les dNTPs utilisables pour une réaction de PCR sont en général conservés sous une forme cristallisée (ou séchée), puis réhydratés et ajoutés au mélange réactionnel au moment de la mise en oeuvre de la réaction PCR.

Les brevets américains US 5,861,251 et US 6,153,412 décrivent la préparation d'une composition lyophilisée formulée comme mélange « prêt à l'emploi » pour la mise en oeuvre de réactions PCR, comprenant l'utilisation d'un agent stabilisant, une ADN polymérase, des dNTPs et du chlorure de magnésium, préalablement à l'étape de lyophilisation. Parmi ces agents stabilisants, sont cités la gélatine, la BSA, le Thesit, le PEG8000 et les polyols, tels que le ficoll, le sucrose, le glycérol, le glucose, le mannitol, le galacitol, le glucitol et le sorbitol.

Le tréhalose est également cité comme agent stabilisant d'une composition lyophilisés formulée comme mélange « prêt à l'emploi » pour la mise en oeuvre de réactions RT-PCR (US 5,832,254), et plus généralement pour la lyophilisation ou la déshydratation de macromolécules biologiques, plus particulièrement des protéines (US 5,834,254).

Le brevet américain US 5,310,885 et la demande de brevet US 2002/064536 décrivent le collagène et les polysaccharides tels que le saccharose et le tréhalose comme agents stabilisants des protéines, sans toutefois décrire l'utilisation combinée du collagène et d'un disaccharide, ni mentionner spécifiquement les protéines pour la mise en oeuvre de réactions PCR.

Afin de maintenir la structure tri-dimensionnelle des protéines lorsque celles-ci sont dans des conditions d'environnement facilitant leur dénaturation, on a recours également à différents agents stabilisants et notamment des protéines telles que l'albumine ou le collagène. Le collagène est notamment connu comme agent stabilisant de protéines, notamment de protéines lyophilisées.

Les techniques d'amplification d'acides nucléiques sont utilisées dans de nombreux domaines, notamment en agro-alimentaire ou en agriculture, pour le diagnostic médical, pour la mise en oeuvre de tests génétiques, en archéologie, en criminologie.

Compte tenu de l'utilisation croissante de ces techniques dans des domaines très variés, il existe un réel besoin de développer des kits pour la mise en oeuvre de ces méthodes qui puissent être conservés à température ambiante, sur de longues périodes.

En outre, afin de réduire les risques de contamination lors de la mise en oeuvre de la réaction et la préparation des mélanges réactionnels, des mélanges « prêt à l'emploi » contenant une majorité de réactifs pré-aliquotés dans les quantités appropriées sont plus particulièrement recherchés.

L'aspect des compositions lyophilisées est un facteur important dans le cadre d'une commercialisation de ces compositions, notamment pour la mise en oeuvre de réactions de biologie moléculaire telles que la PCR. En effet, une présentation des compositions lyophilisées sous une forme compacte et dense est généralement considérée par l'homme du métier comme un gage de bonne qualité.

La présente invention résulte en particulier de la constatation que la lyophilisation de compositions d'acides nucléiques en présence d'une combinaison de disaccharides et de collagène permet d'améliorer très significativement le temps de conservation à température ambiante des acides nucléiques et notamment des oligonucléotides ou de mélanges de dNTPs.

La présente invention résulte également de la constatation que la lyophilisation de compositions d'acides nucléiques en présence d'une combinaison de disaccharides et de collagène permet d'obtenir un aspect compact et dense des compositions, tel que recherché dans le cadre d'une commercialisation.

A la connaissance de la demanderesse, l'utilisation combinée de tréhalose et de collagène comme agent de stabilisation et/ou de compaction d'acides nucléiques lyophilisés n'a jamais été décrite ni suggérée dans l'état de la technique.

Au sens de l'invention, le terme « nucléotide » signifie, sauf précision spécifique, une molécule constituée d'une base azotée (généralement l'adénine, la thymine ou l'uracile, la cytosine et la guanine), un sucre (ribose ou désoxyribose) et un ou plusieurs groupes phosphate. Un nucléotide est notamment un ribonucléoside-5'-triphosphate ou un désoxyribonucléoside-5'-triphosphate et leurs dérivés qui peuvent servir de substrats pour les ADN polymérase ou les ARN polymérase. En particulier il s'agit du dATP, dGTP, dlTP, dUTP, dCTP, dTTP, ou de l'ATP, GTP, ITP, UTP, CTP, TTP.

Le terme «dNTPs» signifie, au sens de l'invention, un mélange équimolaire de dATP, dCTP, dGTP et dTTP.

Le terme « polynucléotide » signifie, au sens de l'invention, sauf précision spécifique, un polymère de nucléotides comprenant au moins 50 nucléotides, simple brin ou double brin, ADN ou ARN notamment.

Le terme « oligonucléotide » signifie, au sens de l'invention, sauf précision spécifique, un polymère de nucléotides simple brin comprenant au plus 50 nucléotides, de préférence de 10 à 30 nucléotides.

Le terme acide nucléique fait référence indifféremment aux nucléotides, aux oligonucléotides ou aux polynucléotides simple brin ou double brins.

La présente invention concerne en particulier une méthode d'obtention d'une composition lyophilisée contenant des acides nucléiques, caractérisée en ce qu'elle comprend la lyophilisation d'une solution aqueuse d'acides nucléiques en présence de disaccharides et de collagène.

Tout disaccharide et leurs combinaisons peuvent être utilisés dans la méthode de l'invention. On citera en particulier le saccharose, le maltose, le lactose et le tréhalose. Dans un mode de réalisation particulier, on utilisera le tréhalose.

Le collagène utilisé dans la méthode de l'invention peut être par exemple un collagène obtenu par hydrolyse thermique de collagène de derme d'un mammifère, et notamment un collagène obtenu par hydrolyse thermique à 145°C de collagène de derme de porc, hautement purifié.

Les quantités de disaccharides et de collagène seront optimisées par l'homme du métier de sorte à obtenir la meilleure stabilité possible des acides nucléiques. La stabilité des acides nucléiques peut être mesurée par exemple par comparaison de la sensibilité d'une réaction d'amplification d'acides nucléiques en présence des acides nucléiques testés après une période de conservation déterminée et des acides nucléiques contrôle préparé extemporanément.

Dans un mode de mise en oeuvre particulier, la solution aqueuse comprend, 800 µM d'acides nucléiques (4 X 200 µM de dNTP), de 150 mM à 600 mM de disaccharides, et de 0,1% à 1% de collagène.

La solution aqueuse peut comprendre au moins l'un des acides nucléiques suivants :
- un 2'désoxyribonucléoside-5'-triphosphate;
- un oligonucléotide ;
- un polynucléotide.

Les acides nucléiques de faible poids moléculaire sont plus particulièrement instables. La présente invention est appropriée pour fournir des compositions contenant des acides nucléiques de faible poids moléculaire stabilisés par lyophilisation en présence de disaccharides et de collagène.

Dans un mode de réalisation particulier de la méthode selon l'invention, les acides nucléiques contenus dans la solution aqueuse sont des oligonucléotides ou des nucléotides, et notamment des dNTPs.

La méthode de l'invention permet en particulier d'obtenir des compositions lyophilisées pour la mise en oeuvre de réactions de biologie moléculaire, comprenant des acides nucléiques et tout autre élément utile dans la mise en oeuvre d'une réaction de biologie moléculaire. Parmi ces éléments, on citera les enzymes utilisant comme substrat ou co-substrat des acides nucléiques, les tampons de réaction, les agents stabilisants ou cofacteurs d'enzymes, les agents réducteurs et les colorants.

Parmi les enzymes utilisant comme substrat ou co-substrat des acides nucléiques, on citera à titre d'exemples, les ADN polymérases et notamment les ADN polymérases thermostables, les ADN polymérases sur matrice d'ARN (transcriptase inverse), les ARN polymérase et les enzymes à activité correctrice 5'-3'.

Parmi les agents stabilisants ou cofacteurs, on citera en particulier les sels de métaux tels que les sels de zinc ou de magnésium, les protéines, les disaccharides ou encore le polyvinylpyrrolidone (PVP).

Parmi les colorants, on citera par exemple le bleu de bromophénol, le cyanole de xylène, le rouge de bromocresol ou le rouge de cresol.

Selon les applications souhaitées, l'homme du métier choisira, parmi les composants décrits ci-dessus, les composants à ajouter dans la solution aqueuse avant la lyophilisation, dans les quantités appropriées pour que l'application puisse être mise en oeuvre directement après réhydratation de la composition lyophilisée ou à tout le moins, en minimisant la quantité de composants à ajouter. -

La présente invention vise en particulier l'obtention de compositions formulées comme mélanges « prêt à l'emploi » pour l'amplification d'acides nucléiques par réaction de polymérisation en chaîne (PCR). Pour la préparation de tels mélanges, la solution aqueuse préparée avant lyophilisation comprend par exemple, en plus du tréhalose et du collagène, les composants suivants :
- un mélange de 2'déoxyribonucléoside-5'-phosphates (dNTPs) ;
- un tampon de réaction ;et
- le cas échéant, un oligonucléotide ; du chlorure de magnésium ; une ADN polymérase ; une enzyme à activité correctrice 5'-3' et/ou un colorant.

Le terme « prêt à l'emploi » signifie dans le texte que les composants du mélange sont présents dans des quantités appropriées pour la mise en oeuvre d'une réaction d'amplification d'acides nucléiques par réaction de polymérisation en chaîne (PCR) après réhydratation sans modifier les quantités respectives de chacun des composants présents initialement dans le mélange lyophilisé. Bien entendu, pour la mise en oeuvre de la réaction, l'homme du métier devra le cas échéant ajouter certains composants non présents initialement dans le mélange lyophilisé.

Dans un mode de réalisation particulier, une telle composition selon l'invention ne comprend pas d'enzymes, en particulier, ne comprend pas d'ADN polymérase.

Un exemple de solution aqueuse en vue de la préparation de mélanges lyophilisés « prêt à l'emploi » pour l'amplification d'acides nucléiques par PCR est une solution comprenant, outre le tréhalose et le glycogène,
- un mélange de 2'déoxyribonucléoside-5'-phosphates (dNTPs) ;
- un tampon de réaction ;
- un ou plusieurs couples d'amorces, chaque couple d'amorces permettant l'amplification d'une séquence d'acides nucléiques particulière ;
- une ADN polymérase ; et
- du chlorure de magnésium.

Il est bien connu que la conservation et la lyophilisation d'acides nucléiques sont d'autant moins efficaces que les quantités lyophilisées sont réduites. Or, dans la majorité des applications de mélange lyophilisé « prêt à l'emploi », les quantités d'acides nucléiques, notamment de dNTPs ou d'oligonucléotides sont inférieures à 3000 µM. Un des avantages de la présente invention est qu'elle permet de lyophiliser et de conserver sous une forme lyophilisée de très petites quantités d'acides nucléiques.

Dans un mode de mise en oeuvre de la méthode, la solution aqueuse contient une quantité d'acide nucléique inférieure à 3000 µM, de préférence comprise entre 600 µM et 1000 µM et dans la majorité des applications une quantité de 800 µM (4 X 200 µM dNTP).

Selon la méthode de l'invention, la lyophilisation consiste à congeler la solution, puis à sublimer l'eau par réchauffement sous vide de manière à éliminer l'eau et ne garder que les produits déshydratés. Les techniques de lyophilisation sont bien connues de l'homme du métier. Un exemple de protocole de lyophilisation est en outre décrit dans la partie expérimentale. Dans un mode de réalisation particulier, les paramètres suivants sont compris dans les plages indiquées ci-après :
Température de congélation : entre - 20°C et - 50°C
La température des plateaux : entre 0°C et 30°C
La température du piège : entre - 10°C et - 60°C
La valeur de vide : entre 0,1 et 0,01 mbar
Et l'arrêt de la lyophilisation 3 à 10 heures après stabilisation de la température du produit lyophilisé entre 10°C et 40°C.

Les valeurs de ces paramètres peuvent être adaptées selon le type de solution à lyophiliser.

La présente inventive vise naturellement une composition lyophilisée contenant des acides nucléiques, caractérisée en ce qu'elle comprend des disaccharides et du collagène, par exemple du tréhalose et du collagène.

De telles compositions sont notamment celles susceptibles d'être obtenues par la méthode décrite ci-dessus.

Les compositions lyophilisées selon l'invention comprennent en général de 5 à 50 mmoles de disaccharides et 40 à 400 µg de collagène.

Dans un mode de réalisation particulier, une composition lyophilisée selon l'invention comprend une quantité d'acide nucléique inférieure à 100 µmoles, de préférence comprise entre 20 µmoles et 30 µmoles.

A titre d'exemple, un acide nucléique compris dans la composition lyophilisée selon l'invention est un 2'désoxyribonucléoside-5'-phosphates; un oligonucléotide ; et/ou un polynucléotide, en particulier un mélange de dNTPs ou un ou plusieurs oligonucléotide(s).

Dans un mode de réalisation particulier, les compositions selon l'invention sont formulées comme mélange « prêt à l'emploi » pour le séquençage de molécules d'ADN selon la méthode de Sanger (Sanger, 1977). Une telle composition lyophilisée comprend, outre le disaccharide et le collagène, des dNTPs et des didésoribonucléoside-5'-triphosphate (ddNTPs).

Dans un autre mode de réalisation particulier, la composition lyophilisée selon l'invention est un mélange lyophilisé « prêt à l'emploi » pour la mise en oeuvre de réaction PCR, comprenant, en plus du disaccharide et du collagène, au moins les composants suivants :
- un mélange de 2'déoxyribonucléoside-5'-phosphates (dNTPs) ;
- un tampon ;et
- le cas échéant, un oligonucléotide ;du chlorure de magnésium ; une ADN polymérase ; et/ou un colorant ;

Par exemple, un mélange lyophilisé « prêt à l'emploi » selon l'invention pour l'amplification d'acides nucléiques par PCR est une composition lyophilisée comprenant :
- des disaccharides de 5 à 50 mmoles
- 40 à 400 µg de collagène
- un ou plusieurs couples d'oligonucléotides de 4 µmoles à 20 µmoles
- des dNTPs de 20 à 30 µmoles
- une ADN polymérase : 0,1 à 5 unités

La réaction de PCR pourra être mise en oeuvre après réhydratation de la composition lyophilisée définie ci-dessus en ajoutant une solution susceptible de contenir en particulier la séquence d'acide nucléique à amplifier.

Dans un mode de réalisation particulier de la composition définie ci-dessus, les quantités de chacun de ces composants sont appropriées pour la mise en oeuvre d'une réaction d'amplification d'acides nucléiques par PCR après réhydratation de la composition dans un volume d'eau ou d'une solution appropriée pour la mise en oeuvre de réaction PCR, et notamment d'un tampon approprié, compris entre 10 et 100 µL. Les compositions lyophilisées selon l'invention peuvent être contenues dans un microtube utilisable dans un thermocycleur pour les réactions de PCR.

En général, un microtube utilisable dans un thermocycleur pour les réactions de PCR, est un microtube en polypropylène à parois fines susceptible de contenir un volume réactionnel compris entre 50 µl et 200 µl, de préférence de 50 µl à 100 µl.

L'invention concerne également un procédé d'amplification d'acide nucléique, comprenant la réhydratation d'une composition lyophilisée formulée comme mélange « prêt à l'emploi » pour la mise en oeuvre de réaction PCR telle que définie ci-dessus, par l'ajout d'une solution aqueuse comprenant le cas échéant un ou plusieurs composants nécessaires à l'amplification d'acides nucléiques par PCR et en particulier une matrice d'ADN et/ou un couple d'oligonucléotides.

L'invention vise également toute utilisation d'une combinaison de disaccharides et de collagène comme agent de stabilisation et/ou de compaction d'acides nucléiques lyophilisés, en particulier d'une combinaison de tréhalose et de collagène.

La présente invention et ses avantages sont illustrées par les exemples suivants.

### DESCRIPTION DES FIGURES

**Figure 1** : La figure 1 illustre le protocole de lyophilisation.

| | | |
|---|---|---|
| Courbe A : | 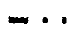 | température du produit lyophilisé |
| Courbe B : | 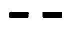 | température des plateaux |
| Courbe C : | 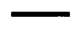 | température de piège |
| Courbe D : | 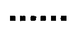 | valeur de vide |

**Figure 2 :** Aspect des lyophilisats

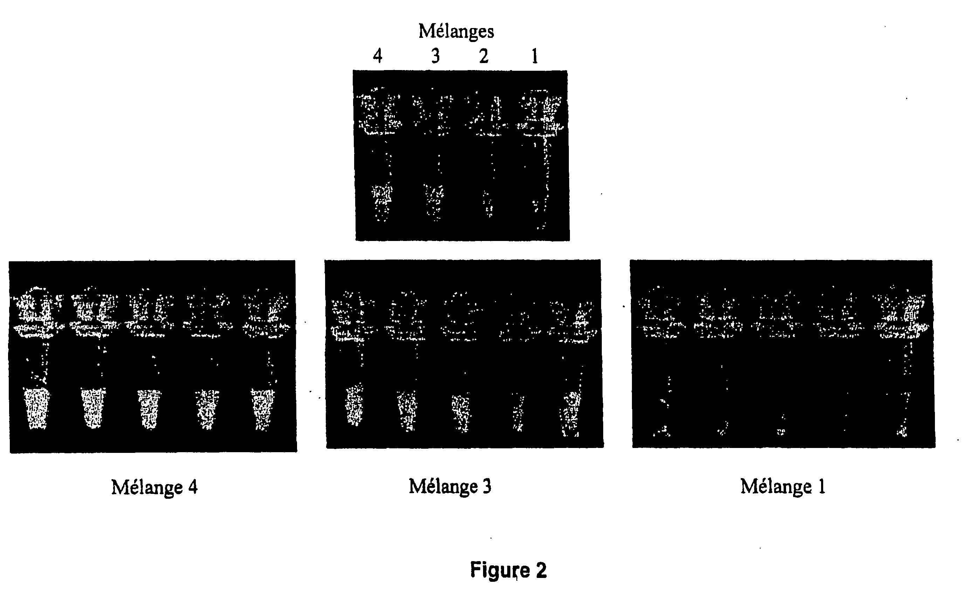

La figure 2 illustre l'aspect des mélanges obtenus après lyophilisation
- en absence de conservateur (mélange 1)
- en présence de tréhalose (mélange 2)
- en présence de collagène (mélange 3)
- en présence de tréhalose et de collagène (mélange 4).

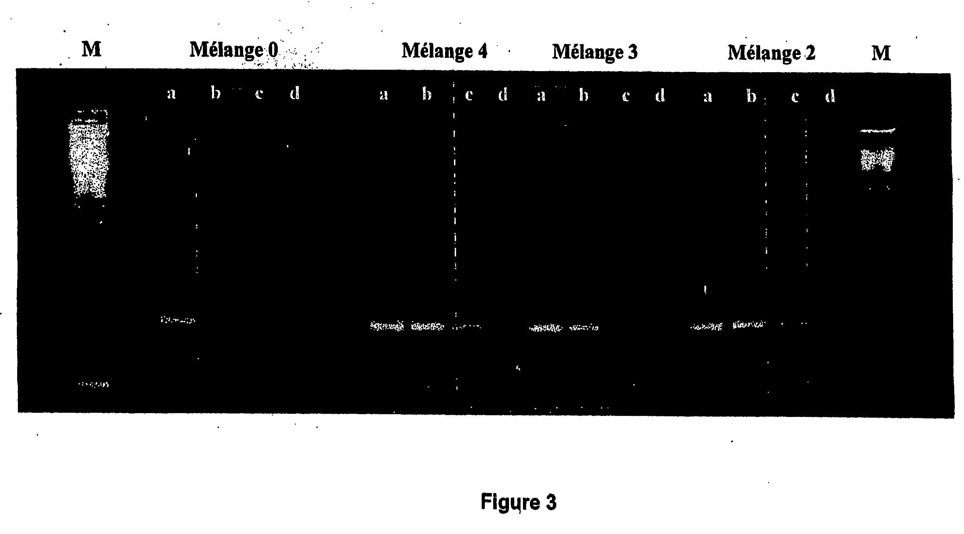

**Figure 3 :** Electrophorèse des amplificons d'ADN de *Bordetella pertussis* obtenus par PCR à partir de :
- 1000 bactéries: = piste a
- 100 bactéries: = piste b
- 10 bactéries: = piste c
- 1 bactérie: = piste d

- Mélange 1 :: Produit non lyophilisé contenant les éléments constituant le tampon de réaction, les dNTPs, l'ADN polymérase et les amorces.
- Mélange 2 :: Produit lyophilisé dans des microtubes contenant les éléments constituant le tampon de réaction, les dNTPs, l'ADN polymérase, les amorces et le tréhalose.
- Mélange 3 :: Produit lyophilisé dans des microtubes contenant les éléments constituant le tampon de réaction, les dNTPs, l'ADN polymérase, les amorces et le Prionex^{R} (collagène obtenu par hydrolyse thermique de collagène de derme de porc, hautement purifié).
- Mélange 4 :: Produit lyophilisé dans des microtubes contenant les éléments constituant le tampon de réaction, les dNTPs, l'ADN polymérase, les amorces, le tréhalose et le Prionex^{R}.

### EXEMPLES

### Exemple 1 : Préparation d'une composition lyophilisée formulée comme mélange « prêt à l'emploi »

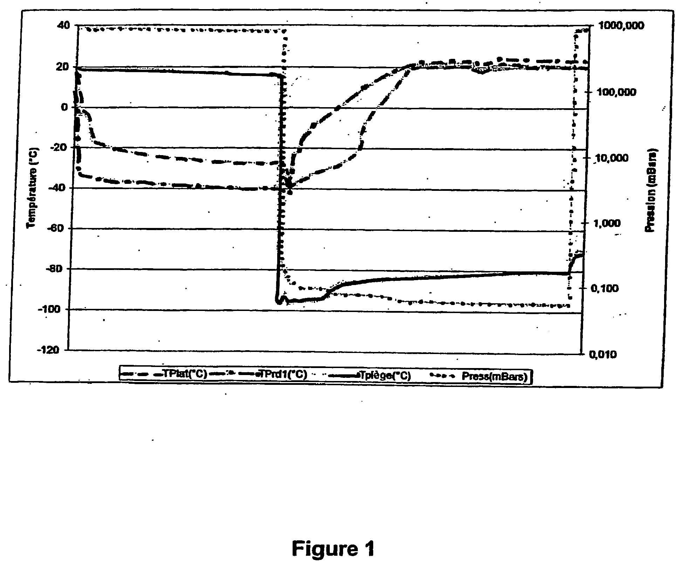

Dans un microtube [par PCR de 200 µL], on dépose 40 µL d'une solution aqueuse contenant :
Tampon de réaction : Tris HCl (10 mM - pH 8,4); KCl (50 mM); MgCl₂ (1,5 mM)
Mélange dNTPs : 200 µM de chaque
ADN polymérase : 2,5 unités (Hotstart Taq)
Amorces (facultatif): 1 µM
Disaccharides : 300 mM
Collagène 0,5%
La lyophilisation est effectuée selon le protocole suivant (tel qu'illustré à la figure 1) :
   - une congélation progressive des compositions jusqu'à -20°C
   - un chauffage des plateaux à 20°C
   - une température du piège à -50°C
   - une valeur de vide de 0,05 mbar
   - un arrêt de la lyophilisation lorsque la température des produits atteint 20°C.

La composition ainsi lyophilisée peut être conservée à température ambiante jusqu'à au moins trois mois sans perte d'activité.

Pour la mise en oeuvre de la réaction PCR, la composition est réhydratée avec 45µl d'eau et 5 µL de matrice d'ADN et le cas échéant les amorces à une concentration finale de 1 µM.

La réaction PCR est mise en oeuvre à l'aide d'un thermocycleur Perkins et comprend :
- 95°C 15 mn: 1 cycle 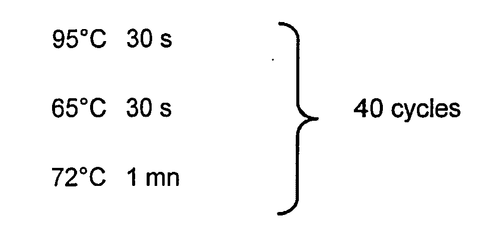
- 72°C 10 m: 1 cycle
- 8°c Infini:

### Exemple 2 : Composition lyophilisée contenant des dNTPs

Un mélange lyophilisé de dNTPs (dATP, dCTP, dGTP et dTTP) est préparé par lyophilisation d'un mL d'une solution aqueuse comprenant :

| | |
|---|---|
| Tréhalose | 300mM |
| Collagène | 0,5% - |
| dNTPs | 2 mM de chaque dNTP |
| Tampon | Tris HCl (100 mM-pH 8,4) KCI (50 mM) MgCl₂ (15 mM) |

Une réhydratation de cette solution dans un 1 ml d'eau distillée permet d'obtenir une solution de dNTPs à 8 mM prêt à l'emploi. (2 mM de chaque dNTP).

Une telle solution ainsi réhydratée est utilisable en particulier dans un milieu réactionnel de PCR (5 µl dans un milieu réactionnel de PCR final de 50 µl permet d'obtenir une concentration appropriée de 200 µM de chaque dNTP).

Alternativement, il est possible de préparer des compositions lyophilisées pour chacun des dNTPs (dATP, dCTP, dGTP ou dTTP). Des kits comprenant 4 microtubes contenant chacun l'un des 4 dNTPs peuvent être ainsi obtenus.

Ces solutions sont également utilisables pour le marquage d'ADN ou le séquençage.

### Exemple 3 : Exemples de mélanges « prêt à l'emploi » pour la mise en oeuvre de réaction PCR

Un mélange « prêt à l'emploi » peut être obtenu par lyophilisation selon les conditions décrites à l'exemple 1 d'un volume de 40 µL d'une solution aqueuse suivante :

### Mélange PCR

**Tampon de réaction : Tris HCl (10mM pH 8,4) KCI (5mM) MgCl₂ (1,5mM)**

| | |
|---|---|
| dNTPs | 200 µM de chaque |
| ADN polymérase | 2,5 unités (Hotstart Taq) |
| Amorces | 1 µM |
| DMSO | facultatif si oui 5% |
| Collagène | 0,5% |
| Tréhalose | 300mM |

### Exemple 4 : Mélange « prêt à l'emploi » pour la mise en oeuvre d'une réaction de réverse transcription.

Les mélanges « prêt à l'emploi » sont obtenus par lyophilisation selon les conditions décrites à l'exemple 1 d'un volume de 40 µL d'une solution aqueuse suivante :

| | |
|---|---|
| Tampon de réaction : | Tris HCl (10 mM) KCI (5 mM) MgCl₂ (2 mM) |
| Amorce sens: | 1 µM |
| Amorces antisens : | 1 µM |
| dNTPs : | 200 µM de chaque |
| Réverse transcriptase : | MMLV RT Biolabs 10 unités |
| lnhibiteur(s) de RNase : | RNAsine 10 unités |
| Taq polymerase Promega : | 0,02 unité |

La composition est réhydratée avec 50 µL d'eau distillée contenant les ARNm et le cas échéant des amorces. Elle permet la génération d'ADNc pour le clonage et l'analyse et/ou la mesure du niveau d'expression d'un gène par détection de l'ARNm correspondant après amplification quantitative (RT-PCR quantitative).

### Exemples comparatifs :

Afin de mettre en évidence les avantages de l'utilisation d'une combinaison de disaccharides et de Prionex^{R}, une étude comparative a été réalisée sur des mélanges pour l'amplification d'un fragment d'ADN de *Bordetella pertussis.*

### A - Les mélanges suivants ont été testés :

- Mélange 0 :: Solution contenant :
- les éléments constituant le tampon de réaction, Tris HCl (10 mM) KCl (5 mM) MgCl₂ (2 mM)
- les dNTPs : 200 µM de chaque
- l'ADN polymérase (Hot start Taq) 2,5 unités
- les amorces :
- Amorce 1 = 5' ccaacgcgcatgcgtgcagattcgtc 3'
- Amorce 2 = 5' ccctctgcgttttgatggtgcctatttta 3'
- Mélange 1 :: Produit lyophilisé dans des microtubes sous un volume de 40µl et contenant :
- les éléments constituant le tampon de réaction, Tris HCl (10 mM) KCl (5 mM) MgCl₂ (2 mM)
- les dNTPs, 200 µM de chaque
- l'ADN polymérase (Hot start Taq) 2,5 unités
- les amorces.
   Amorce 1 = 5' ccaacgcgcatgcgtgcagattcgtc 3'
   Amorce 2 = 5' ccctctgcgttttgatggtgcctatttta 3'
- Mélange 2 :: Produit lyophilisé dans des microtubes sous un volume de 40µl et contenant :
- les éléments constituant le tampon de réaction, Tris HCl (10mM) KCl (5mM) MgCl₂ (2mM)
- les dNTPs, 200 µM de chaque
- l'ADN polymérase (Hot start Taq) 2,5 unités
- les amorces
   Amorce 1 = 5' ccaacgcgcatgcgtgcagattcgtc 3'
   Amorce 2 = 5' ccctctgcgttttgatggtgcctatttta 3'
- le tréhalose 300 mM
- Mélange 3: Produit lyophilisé dans des microtubes sous un volume de 40µl et contenant :
- les éléments constituant le tampon de réaction,Tris HCl (10 mM) KCl (5 mM) MgCl₂ (2 mM)
- les dNTPs, 200 µM de chaque
- l'ADN polymérase (Hot start Taq) 2,5 unités
- les amorces
   Amorce 1 = 5' ccaacgcgcatgcgtgcagattcgtc 3'
   Amorce 2 = 5' ccctctgcgttttgatggtgcctatttta 3'
- le Prionex^{R} 0,5%
- Mélange 4 :: Produit lyophilisé dans des microtubes sous un volume de 40µl et contenant :
- les éléments constituant le tampon de réaction, Tris HCI (10 mM) KCl (5 mM) MgCl₂ (2 mM)
- les dNTPs, 200 µM de chaque
- l'ADN polymérase (Hot start Taq) 2,5 unités
- les amorces
   Amorce 1 = 5' ccaacgcgcatgcgtgcagattcgtc 3'
   Amorce 2 = 5' ccctctgcgttttgatggtgcctatttta 3'
- le tréhalose 300 mM
- le Prionex^{R} 0,5%

### B - Les protocoles

**(i) Protocole pour l'étude de la réhydratation « spontanée » :**
   1) conserver les tubes contenant les produits lyophilisés à la température ambiante (18 à 22°C) pendant 120 jours ;
   2) observer les lyophilisats tous les 2 ou 3 jours et noter leur aspect.
**(ii) Protocole pour l'étude de la sensibilité :**
   1) préparer des suspensions de bactéries de façon à avoir 10³, 10⁴, 10⁵ ou 10⁶ bactéries par ml, soit 1, 10, 100 ou 1000 dans 10 µl ;
   2) préparer les échantillons d'ADN à partir des suspensions de bactéries ;
   3) pour l'amplification, déposer 90 µl du mélange 0 préparé extemporanément dans des microtubes;
   4) reprendre les mélanges lyophilisés 1, 2, 3 et 4 avec 90 µl d'eau distillée (ou équivalent);
   5) ajouter dans tous les tubes 10 µl de la matrice d'ADN à amplifier ;
   6) après amplification, analyser les produits par électrophorèse et noter la présence et l'intensité des bandes à l'aide des indications suivantes :
      - absence de bande -
      - bande de faible intensité +
      - bande de forte intensité ++ à ++++.
**(iii) Protocole pour l'étude de la stabilité :**
   1) conserver le mélange 0 et les mélanges lyophilisés à 37°C pendant 120 jours ;
   2) préparer des suspensions de bactéries de façon à avoir 10³, 10⁴, 10⁵ ou 10⁶ bactéries par ml, soit 1, 10, 100 ou 1000 dans 10 µl ;
   3) préparer les échantillons d'ADN à partir des suspensions de bactéries ;
   4) pour l'amplification, déposer 90 µl du mélange 1 préparé extemporanément dans des microtubes;
   5) reprendre les mélanges lyophilisés 1, 2, 3 et 4 avec 90 µl d'eau distillée;
   6) ajouter dans tous les tubes 10 µl de la matrice d'ADN à amplifier;
   7) après amplification, analyser les produits par électrophorèse et noter la présence et l'intensité des bandes.

### C - Résultats

### 1. Aspect des lyophilisats et réhydratation

La figure 2 illustre l'aspect des produits obtenus après lyophilisation en absence de conservateur (mélange 1) ou en présence de tréhalose (mélange 2) ou de Prionex^{R} (mélange 3) ou en présence des deux conservateurs (mélange 4). Sur le plan présentation d'un produit commercialisable, le meilleur aspect (lyophilisat compact et dense) est obtenu lorsque la lyophilisation est réalisée en présence du tréhalose et du Prionex^{R}. Une réhydratation des lyophilisats, lors d'une conservation à la température du laboratoire (18-22°C) est observée en 3 à 6 jours pour les lyophilisats ne contenant aucun conservateur (mélange 1) ou ne contenant que du tréhalose (mélange 2). Aucune réhydratation n'est observée pour les lyophilisats contenant du Prionex^{R} seul (mélange 3) ou du tréhalose et du Prionex^{R} (mélange 4) après une conservation pendant 120 jours.

### 2. Sensibilité

La figure 3 montre que le mélange 4 contenant du tréhalose et du Prionex^{R} permet d'obtenir une sensibilité au moins aussi élevée que celle obtenue avec le mélange 0 (1 à 10 bactéries).

### 3. Stabilité des produits

On constate (tableau 1) pour les produits non lyophilisés (mélange 0) ou lyophilisés en absence de conservateur (mélange 1) ou en présence de Prionex^{R} (mélange 3) une perte d'activité après une conservation supérieure à 7 jours à 37°C.

En présence de tréhalose (mélange 2), une perte d'activité est observée après 30 jours à 37°C. Par contre, les produits lyophilisés en présence de tréhalose et de Prionex^{R} (mélange 4) conservent leur activité pendant au moins 120 jours.

**Tableau 1 : Stabilité des lyophilisats après conservation (J0 à J120) à 37°C.**

| Temps (en jours) de conservation à 37°C | Quantité de bactéries détectées après amplification avec les différents mélanges | | | | |
|---|---|---|---|---|---|
| | **Mélange 0** | **Mélange 1** | **Mélange 2** | **Mélange 3** | **Mélange 4** |
| J 0 | 1-10 | 1-10 | 1-10 | 1-10 | 1-10 |
| J 7 | 1-10 | 1-10 | 1-10 | 1-10 | 1-10 |
| J 30 | 10-100 | 10-100 | 1-10 | 10-100 | 1-10 |
| J60 | 10-100 | 10-100 | 10-100 | 10-100 | 1-10 |
| J 90 | > 1000 | > 1000 | 10-100 | 100-1000 | 1-10 |
| J 102 | > 1000 | > 1000 | 100-1000 | 100-1000 | 1-10 |

Les résultats présentés ci-dessus montrent que les compositions contenant à la fois un disaccharide (le tréhalose) et du collagène
(i) présentent un bel aspect compact et dense ;
(ii) ne se réhydratent pas spontanément, et
(iii) ont une excellente stabilité dans le temps.

La présence concomitante du tréhalose et du collagène dans les mélanges « prêt à l'emploi » lyophilisés, formulés pour l'amplification des acides nucléiques, permet une meilleure stabilisation et une meilleure présentation du produit tout en conservant une bonne sensibilité de la réaction.

### Exemple 5 : Etudes d'amplification d'ADN de Bordetella pertussis réalisée à partir de mélanges lyophilisés formulés pour la mise en oeuvre de réaction PCR et contenant du collagène en présence de différents oses (saccharose, glucose, lactose et tréhalose).

Les mélanges lyophilisés contiennent :
- les éléments constituant le tampon de réaction, Tris HCl (10 mM) KCl (5 mM) MgCl₂ (2 mM)
- les dNTPs, 200 µM de chaque
- l'ADN polymérase (Hot start Taq) 2,5 unités
- les amorces
   Amorce 1 = 5' ccaacgcgcatgcgtgcagattcgtc 3'
   Amorce 2 = 5' ccctctgcgttttgatggtgcctatttta 3'
   Le Prionex^{R} à 0,5%
   L'ose à 300 mM
   Le mélange 1 contient du saccharose
   Le mélange 2 contient du glucose
   Le mélange 3 contient du lactose
   Le mélange 4 contient du maltose
   Le mélange 5 contient du tréhalose

**Résultats de l'amplification d'ADN de Bordetella pertissis**

| | Quantité de bactéries détectées après amplification avec les différents mélanges | | | | |
|---|---|---|---|---|---|
| Sensibilite | Mélange 1 saccharose | Mélange 2 Glucose | Mélange 3 Lactose | Mélange 4 Maltose | Mélange 5 Tréhalose |
| à J0 | 1 à 10 | 10 à 100 | 1 à 10 | 1 à 10 | 1 à 10 |
| Après 30 jours à 37°C | 1 à 10 | 10 à 100 | 1 à 10 | 1 à 10 | 1 à 10 |
| Après 60 jours à 37°C | 10 à 100 | 100 à 1000 | 10 à 100 | 10 à 100 | 1 à 10 |

Les résultats obtenus démontrent la supériorité du tréhalose en terme de conservateur par rapport aux autres oses.

## Revendications

1. Méthode d'obtention d'une composition lyophilisée contenant des acides nucléiques, **caractérisée en ce qu'**elle comprend la lyophilisation d'une solution aqueuse d'acides nucléiques en présence de disaccharides et de collagène.

2. Méthode selon la revendication 1, **caractérisée en ce que** le disaccharide est le tréhalose.

3. Méthode selon l'une des revendications 1 ou 2, **caractérisée en ce que** la solution aqueuse comprend de 150 à 600 mM de disaccharides et de 0,1 à 1 % de collagène.

4. Méthode selon l'une quelconque des revendications 1 à 3, **caractérisée en ce que** la solution aqueuse comprend au moins l'un des acides nucléiques suivants :
- un 2'déoxyribonucléoside-5'-phosphates;
- un oligonucléotide ;
- un polynucléotide.

5. Méthode selon l'une quelconque des revendications 1 à 4, **caractérisée en ce que** la solution aqueuse comprend, en plus des disaccharides et du collagène, les composants suivants :
- un mélange de 2'déoxyribonucléoside-5'-phosphates (dNTPs) ;
- un tampon de réaction ;et
- le cas échéant, un oligonucléotide ;du chlorure de magnésium ; une ADN polymérase ; et/ou un colorant ;
lesdits composants étant présents dans des quantités appropriées pour la mise en oeuvre d'une réaction d'amplification d'acides nucléiques par réaction de polymérisation en chaîne (PCR) après réhydratation de la composition lyophilisée.

6. Méthode selon l'une quelconque des revendications 1 à 5, **caractérisée en ce que** la solution aqueuse contient une quantité d'acide nucléique inférieure à 3000 µM, de préférence comprise entre 600 et 1000 µM.

7. Composition lyophilisée contenant des acides nucléiques, **caractérisée en ce qu'**elle est susceptible d'être obtenue par une méthode selon l'une quelconque des revendications 1 à 6.

8. Composition lyophilisée contenant des acides nucléiques, **caractérisée en ce qu'**elle comprend des disaccharides, par exemple du tréhalose, et du collagène.

9. Composition selon la revendication 8, **caractérisée en ce qu'**elle comprend de 5 à 50 mmoles de disaccharides et 40 à 400 µg de collagène.

10. Composition lyophilisée selon l'une quelconque des revendications 8 ou 9, **caractérisée en ce qu'**elle comprend une quantité d'acide nucléique inférieure 100 µmoles, de préférence comprise entre 20 et 30 µmoles.

11. Composition lyophilisée selon l'une quelconque des revendications 8 à 10, **caractérisée en ce qu'**elle comprend au moins l'un des acides nucléiques suivants :
- un 2'déoxyribonucléoside-5'-phosphates;
- un oligonucléotide ; et/ou
- un polynucléotide.

12. Composition selon l'une quelconque des revendications 8 à 11, **caractérisée en ce qu'**elle comprend, en plus des disaccharides et du collagène, au moins les composants suivants :
- un mélange de 2'déoxyribonucléoside-5'-phosphates (dNTPs) ;
- un tampon ;et
- le cas échéant, un oligonucléotide ;du chlorure de magnésium ; une ADN polymérase ; et/ou un colorant;
lesdits composants étant présents dans des quantités appropriées pour la mise en oeuvre d'une réaction d'amplification d'acides nucléiques par réaction de polymérisation en chaîne (PCR) après réhydratation de la composition lyophilisée.

13. Composition selon la revendication 12, **caractérisée en ce que** les quantités de chacun de ces composants sont appropriées pour la mise en oeuvre d'une réaction d'amplification d'acides nucléiques par PCR après réhydratation de la composition dans un volume d'eau compris entre 10 et 100 µl.

14. Composition selon la revendication 13, **caractérisée en ce qu'**elle est contenue dans un microtube utilisable dans un thermocycleur pour les réactions de PCR.

15. Procédé d'amplification d'acide nucléique, comprenant la réhydratation d'une composition lyophilisée selon l'une quelconque des revendications 11 à 14 par l'ajout d'une solution aqueuse comprenant le cas échéant un ou plusieurs composants nécessaires à l'amplification d'acides nucléiques par PCR et en particulier une matrice d'ADN.

16. Utilisation d'une combinaison de disaccharides et de collagène comme agent de stabilisation et/ou de compaction d'acides nucléiques lyophilisés.

17. Utilisation selon la revendication 16, dans laquelle le disaccharide est le tréhalose.

## Claims

1. A method for obtaining a lyophilized composition containing nucleic acids, **characterized in that** it comprises lyophilizing an aqueous solution of nucleic acids in the presence of disaccharides and collagen.

2. A method according to claim 1, **characterized in that** the disaccharide is trehalose.

3. A method according to claim 1 or claim 2, **characterized in that** the aqueous solution comprises 150 mM to 600 mM of disaccharides and 0.1% to 1% of collagen.

4. A method according to any one of claims 1 to 3, **characterized in that** the aqueous solution comprises at least one of the following nucleic acids:
• a 2'-deoxyribonucleoside-5'-phosphate;
• an oligonucleotide;
• a polynucleotide.

5. A method according to any one of claims 1 to 4, **characterized in that**, in addition to disaccharides and collagen, the aqueous solution comprises the following components:
• a mixture of 2'-deoxyribonucleoside-5'-phosphates (dNTPs);
• a reaction buffer; and
• if appropriate, an oligonucleotide; magnesium chloride; a DNA polymerase; and/or a colorant;
said components being present in quantities which are suitable for carrying out a nucleic acid amplification reaction by the polymerase chain reaction (PCR) after rehydrating the lyophilized composition.

6. A method according to any one of claims 1 to 5, **characterized in that** the aqueous solution contains a quantity of nucleic acid which is below 3000 µM, preferably in the range between 600 µM and 1000 µM.

7. A lyophilized composition containing nucleic acids, **characterized in that** it is capable of being obtained by a method according to any one of claims 1 to 6.

8. A lyophilized composition containing nucleic acids, **characterized in that** it comprises disaccharides, for example trehalose, and collagen.

9. A composition according to claim 8, **characterized in that** it comprises 5 to 50 mmoles of disaccharides and 40 to 400 µg of collagen.

10. A lyophilized composition according to claim 8 or 9, **characterized in that** it comprises a quantity of nucleic acid which is below 100 µmoles, preferably in the range between 20 µmoles and 30 µmoles.

11. A lyophilized composition according to any one of claims 8 to 10, **characterized in that** it comprises at least one of the following nucleic acids:
• a 2'-deoxyribonucleoside-5'-phosphate;
• an oligonucleotide; and/or
• a polynucleotide.

12. A composition according to any one of claims 8 to 11, **characterized in that**, in addition to disaccharides and collagen, it comprises at least the following components:
• a mixture of 2'-deoxyribonucleoside-5'-phosphates (dNTPs);
• a buffer; and
• if appropriate, an oligonucleotide; magnesium chloride; a DNA polymerase; and/or a colorant;
said components being present in quantities which are suitable for carrying out a nucleic acid amplification reaction by the polymerase chain reaction (PCR) after rehydrating the lyophilized composition.

13. A composition according to claim 12, **characterized in that** the quantities of each of said components are suitable for carrying out a nucleic acid amplification reaction by PCR after rehydrating the composition in a volume of water in the range between 10 µl and 100 µl.

14. A composition according to claim 13, **characterized in that** it is contained in a microtube that is usable in a thermocycler for the PCR reactions.

15. A nucleic acid amplification method, comprising rehydrating a lyophilized composition according to any one of claims 11 to 14 by adding an aqueous solution comprising, if appropriate, one or more components necessary for the amplification of nucleic acids by PCR, and in particular a DNA matrix.

16. Use of a combination of disaccharides and collagen as an agent for stabilizing and/or compacting lyophilized nucleic acids.

17. Use according to claim 16, in which the disaccharide is trehalose.

## Patentansprüche

1. Verfahren zum Erhalten einer lyophilisierten Zusammensetzung, welche Nukleinsäuren enthält, **dadurch gekennzeichnet, dass** es die Lyophilisierung einer wässrigen Lösung von Nukleinsäuren in Gegenwart von Disacchariden und Collagen aufweist.

2. Verfahren gemäß Anspruch 1, **dadurch gekennzeichnet, dass** das Disaccharid Trehalose ist.

3. Verfahren gemäß Anspruch 1 oder 2, **dadurch gekennzeichnet, dass** die wässrige Lösung 150 bis 600 mM Disaccharide und 0,1 bis 1% Collagen aufweist.

4. Verfahren gemäß einem der Ansprüche 1 bis 3, **dadurch gekennzeichnet, dass** die wässrige Lösung wenigstens eine der folgenden Nukleinsäuren aufweist:
- 2'Desoxyribonukleosid-5'-phosphate;
- ein Oligonukleotid;
- ein Polynukleotid.

5. Verfahren gemäß einem der Ansprüche 1 bis 4, **dadurch gekennzeichnet, dass** die wässrige Lösung zusätzlich zu den Disacchariden und dem Collagen die folgenden Bestandteile aufweist:
- eine Mischung aus 2'Desoxyribonukleosid-5'-phosphaten (dNTPs);
- einen Reaktionspuffer; und
- gegebenenfalls ein Oligonukleotid, Magnesiumchlorid, eine DNA-Polymerase und/oder einen Farbstoff;
wobei die Bestandteile in geeigneten Mengen vorliegen für die Durchführung einer Amplifikationsreaktion von Nukleinsäuren durch PCR- (polymerase chain reaction) Reaktion nach Rehydratisierung der lyophilisierten Zusammensetzung.

6. Verfahren gemäß einem der Ansprüche 1 bis 5, **dadurch gekennzeichnet, dass** die wässrige Lösung eine Menge an Nukleinsäure von weniger als 3000 µM, vorzugsweise zwischen 600 und 1000 µM, enthält.

7. Lyophilisierte Zusammensetzung, welche Nukleinsäuren enthält, **dadurch gekennzeichnet, dass** sie geeignet ist, durch ein Verfahren gemäß einem der Ansprüche 1 bis 6 erhalten zu werden.

8. Lyophilisierte Zusammensetzung, welche Nukleinsäuren enthält, **dadurch gekennzeichnet, dass** sie Disaccharide, zum Beispiel Trehalose, und Collagen aufweist.

9. Zusammensetzung gemäß Anspruch 8, **dadurch gekennzeichnet, dass** diese 5 bis 50 mmol Disaccharide und 40 bis 400 µg Collagen aufweist.

10. Lyophilisierte Zusammensetzung gemäß Anspruch 8 oder 9, **dadurch gekennzeichnet, dass** diese eine Menge an Nukleinsäure von weniger als 100 µmol, vorzugsweise zwischen 20 und 30 µmol, enthält.

11. Lyophilisierte Zusammensetzung gemäß einem der Ansprüche 8 bis 10, **dadurch gekennzeichnet, dass** sie wenigstens eine der folgenden Nukleinsäuren aufweist:
- 2'Desoxyribonukleosid-5'-phosphate;
- ein Oligonukleotid; und/oder
- ein Polynukleotid.

12. Zusammensetzung gemäß einem der Ansprüche 8 bis 11, **dadurch gekennzeichnet, dass** sie zusätzlich zu den Disacchariden und dem Collagen die folgenden Bestandteile aufweist:
- eine Mischung aus 2'Desoxyribonukleosid-5'-phosphaten (dNTPs);
- einen Puffer; und
- gegebenenfalls ein Oligonukleotid, Magnesiumchlorid, eine DNA-Polymerase und/oder einen Farbstoff;
wobei die Bestandteile in geeigneten Mengen vorliegen für die Durchführung einer Amplifikationsreaktion von Nukleinsäuren durch PCR- (polymerase chain reaction) Reaktion nach Rehydratisierung der lyophilisierten Zusammensetzung.

13. Zusammensetzung gemäß Anspruch 12, **dadurch gekennzeichnet, dass** die Menge jedes dieser Bestandteile geeignet ist für die Durchführung einer Amplifikationsreaktion von Nukleinsäuren durch PCR nach Rehydratisierung der Zusammensetzung in einer Menge Wasser von 10 bis 100 µl.

14. Zusammensetzung gemäß Anspruch 13, **dadurch gekennzeichnet, dass** sie in einem Mikroröhrchen enthalten ist, das in einem Thermozykler für PCR-Reaktionen verwendet werden kann.

15. Nukleinsäure-Amplifikationsverfahren, welches Rehydratisierung einer lyophilisierten Zusammensetzung gemäß einem der Ansprüche 11 bis 14 durch Hinzufügen einer wässrigen Lösung mit gegebenenfalls einem oder mehreren für die Amplifikation von Nukleinsäuren durch PCR erforderlichen Bestandteilen, und insbesondere einer DNA-Matrix, aufweist.

16. Verwendung einer Kombination aus Disacchariden und Collagen als Stabilisierungsmittel und/oder Kompaktierungsmittel für lyophilisierte Nukleinsäuren.

17. Verwendung gemäß Anspruch 16, bei welcher das Disaccharid Trehalose ist.
